Europäisches Patentamt

European Patent Office

Office européen des brevets

(19)

(11) Publication number: 0 270 317
A2

(12) **EUROPEAN PATENT APPLICATION**

(21) Application number: 87310505.0

(22) Date of filing: 27.11.87

(51) Int. Cl.⁴: **A61K 45/06** , A61K 31/725 , A61K 31/70 , A61K 31/13 , //(A61K31/725,31:70),(A61K31/-725,31:13)

(30) Priority: 29.11.86 JP 285812/86

(43) Date of publication of application:
08.06.88 Bulletin 88/23

(84) Designated Contracting States:
AT BE CH DE ES FR GB GR IT LI LU NL SE

(71) Applicant: Kabushiki Kaisha Ueno Seiyaku Oyo Kenkyujo
2-31, Koraibashi
Higashi-ku Osaka-shi Osaka-fu(JP)

(72) Inventor: Ueno, Ryuzo
10-27 Nango-cho Nishinomiya-shi
Hyogo-ken(JP)
Inventor: Ueno, Ryuji
2-23 Shimogamomiyazaki-cho Sakyo-ku
Kyoto-shi Kyoto-fu(JP)
Inventor: Kuno, Sachiko
11-4-603, 1-chome Sakasedai
Takarazuka-shi Hyogo-ken(JP)
Inventor: Yoshida, Osamu
Dai-2-shikouryo 7-18 Takatsuka-cho
Nishinomiya-shi Hyogo-ken(JP)

(74) Representative: Atkinson, Peter Birch et al
Marks & Clerk Suite 301 Sunlight House
Quay Street
Manchester M3 3JY(GB)

(54) Treatment of diseases caused by viruses.

(57) A pharmaceutical composition for treating a disease caused by virus comprising

(A) an antiviral agent lacking inhibitory activity (1) against a cell-to-cell infection through cell fusion from a virus infected cell to an uninfected cell and/or (2) against adsorption of a virus to a target cell, and

(B) a compound possessing cell fusion inhibitory activity and/or virus-adsorption inhibitory activity, said antiviral agent and compound being administered in respective amounts so that the combined dosage thereof is effective to treat said disease.

EP 0 270 317 A2

## TREATMENT OF DISEASES CAUSED BY VIRUSES

### BACKGROUND OF THE INVENTION

#### 1. Field of the invention

The present invention relates to treatment, inclusive of prophylaxis and therapy, of diseases caused by viruses. More particularly, this invention provides pharmaceutical composition ( including pharmaceutical composition for veterinary use) containing as an active ingredient a combination of cell-fusion and/or vrius-adsorption inhibitor with an antiviral agent lacking inhibitory activity against cell-to-cell infection through cell fusion from virus infected cells to uninfected cells and/or adsorption of viruses to target cells, which are useful in the treatment of diseases caused by viruses; and a method of treating diseases caused by viruses which comprises administering said combination. The combination as described above can produce antiviral synergism and consequently permit enhancement of antiviral effect, while reducing individual dosages and alleviating adverse side-effects.

Very few chemotherapeutic agents for viral diseases have been available which can exert satisfactory therapeutic effects, in contrast to those for bacterial diseases. The compounds, which are effective against viruses in vitro, often produce severe adverse side-effects when administered to living body, and even antiviral agents that are considered as tolerable for practical use find, in many instances, severely restricted application because of their adverse side-effects. This may be reflected, for example, by such antiviral agents as arabinosyladenine (Ara A) or Acyclovir being in recent years found to be effective against herpesviruses and azidothymidine (AZT) being recently reported to possess effects against AIDS (acquired immunodeficiency syndrome) related viruses (that is to say, human immunodeficiency virus ordinarily referred to briefly as "HIV"); these antiviral agents after being given to man also bring about side-effects such as nausea, emesis, diarrhea, eruption, anemia and phlebitis developed locally at the site of administration, which sometimes leads to forced discontinuation of administration. Under these circumstances, development of antiviral agents with reduced side effects is strongly demanded, while reduction by way of some means of side-effects inherent to the conventional antiviral agents currently constitutes one of the most important research subjects since the latter offers better chance of success.

Viral multiplication is known to proceed through adsorption to and entry into host cells, uncoating, transcription, replication, assembly of particles and budding or release by lysis of cells, and antiviral agents are considered to inhibit either of the above described steps. The present inventors found that sulfuric esters of polysaccharides, or mucopolysaccharides or their sulfated derivatives (hereinafter referred to, in some instances, collectively as "sulfated polysaccharides"), when applied alone to retroviruses, inhibit reverse transcriptase activity derived from retroviruses and also suppress infection to cells with and multiplication in cells of retroviruses (US Patent Application Serial No. 019180).

One of passways for multiplication of AIDS virus, one of retroviruses, has been demonstrated to involve fusion of an AIDS virus infected cell with an uninfected cell, followed by direct penetration of AIDS virus into the uninfected cell. The present inventors have, in the course of extensive research on the mechanism of action provided by the above-described sulfated polysaccharides, discovered that the said sulfated polysaccharides inhibit adsorption of HIV's to target cells and also block direct cell-to-cell infection by HIV through fusion of HIV-infected cells with uninfected cells, thereby displaying capability to suppress multiplication of AIDS virus. Noting that the sulfated polysaccharides manifest quite novel suppressing mechanism for AIDS-virus multiplication through inhibition of virus adsorption and cell-to-cell fusion, in sharp contrast with inhibitory action against reverse transcriptase activity provided by the conventional antiviral agents, the inventors have found furthermore that such two different classes of compounds, when used in combination, can produce significant synergism 20 to 50 times greater than could be expected from simple addition of the individual drug effects. In addition, it has been found that any of the conventional antiviral agents lacking inhibitory activity against cell-to-cell infection through cell fusion from virus-infected cells to uninfected cells and/or adsorption of viruses to target cells, when simply used in combination with any substances possessing cell-fusion and virus-adsorpotion inhibiting activity, can universally achieve such a level of antiviral synergism; in other words, that any such combination constitutes a mechanism of producing antiviral synergism.

The present invention has been completed based on the findings as described above.

## 2. Description of related art

A comprehensive review on antiviral agents is given in Kirk Othmer's Encyclopedia of Chemical Technology, vol. 5, pp. 542-552 (John & Wiley & Sons, New York, USA). Among sulfuric esters of polysaccharides, low-molecularweight dextran sulfates have been commercialized as an antilipemic, anti-arteriosclerotic or anticoagulant agent, while dextran sulfates with relatively high molecular weights are know to exhibit suppressory activity against herpesviruses (European Patent Publication No. 0066379). Since herpesviruses belong to a DNA virus, nevertheless, they are fundamentally different in mode or multiplication from retroviruses depending entirely on reverse transcriptase for synthesis of DNA. Accordingly, to exert effect against herpesviruses does not necessarily mean to be effective against retroviruses. In addition, low-molecular-weight dextran sulfates (having molecular weights or not more than 10,000) are known to be almost ineffective against herpesviruses.

Among muchopolysaccharides or their sulfates, chondroitin sulfate is commercialized as a drug for senorineural hearing impairment, neuralgia, lumbago or chronic nephritis and also in the form of cornea-protective opthalmic solution, while it has been known that keratan sulfate is obtained from cartilages; teichuronic acid from cell walls of Bacillus subtilis; hyaluronic acid from shark skin, whale cartilage or human serum; heparan sulfate from the bovine liver or lung; and chitin from arthropod shells, fungi or yeasts, respectively. A process for producing sulfated derivatives of chondroitin sulfate is described in Japanese Patent Publication (JP, B2) No. 9570/1971.

Heparin is known to inhibit in vitro various enzymes, such as DNA polymerase of phytohemaglutinin stimulated human lymphocytes and reverse transcriptase of simian sarcoma virus (Cancer Research, 38, 2401 to 2407), whereas nothing has been found about whether or not heparin can inhibit infection with retroviruses at the cell level.

## SUMMARY OF THE INVENTION

In the first aspect, the present invention provides a method of treating a disease caused by virus which comprises administering to a subject in need of such treatment

(A) an antiviral agent lacking inhibitory activity (1) against cell-to-cell infection through cell fusion from a virus infected cell to an uninfected cell and/or (2) against adsorption of a virus to a target cell, and

(B) a compound possessing cell fusion inhibitory activity and/or virus-adsorption inhibitory activity, said antiviral agent and compound being administered in respective amounts so that the combined dosage thereof is effective to treat said disease.

In the second aspect, the present invention provides the use of a cell-fusion and/or virus-adsorption inhibitor for the manufacture of a medicament effective for treating diseases caused by viruses, which medicament is specifically intended to be administered in combination with an antiviral agent lacking inhibitory activity against cell-to-cell infection through cell fusion from virus-infected cells to uninfected cells and/or adsorption of viruses to target cells.

In the third aspect, the present invention provides a pharmaceutical composition comprising a synergistically effective amount each of an antiviral agent lacking inhibitory activity against cell-to-cell infection through cell fusion from virus-infected cells to uninfected cells and/or adsorption of viruses to target cells and a cell-fusion and/or virus-adsorption inhibitor.

In the fourth aspect, the present invention provides a method of lowering side effects of an antiviral agent lacking inhibitory activity against cell-to-cell infection through cell fusion from virus-infected cells to uninfected cells and/or adsorption of viruses to target cells, which method comprises administering said antiviral agent in amount lower than usual dose in conjunction with a cell-fusion and/or virus-adsorption inhibitor.

In the fifth aspect, the present invention provides the use of a cell-fusion and/or virus adsorption inhibitor for the maufacture of an antiviral synergistic composition comprising an antiviral agent lacking inhibitory activity against cell-to-cell infection through cell fusion from virus-infected cells to uninfected cells and/or adsorption of viruses to target cells and a cell fusion inhibitor in order to achieve reduction in side effects pertaining to the former antiviral agent.

BRIEF DESCRIPTION OF DRAWING

Figs. 1 and 2 each shows a relationship between a number of viable MT-4 cells and a concentration of dextran sulfate sodium salt (hereinafter referred to briefly as "DS") having a molecular weight of 7,000 to 8,000 and a sulfur content of 17 to 20 %, as was observed when the cells were cultured with HTLV-III viruses in the presence of AZT and/or DS at different concentrations as is to be described in Example 11,

Figs. 3 and 4 each shows a relationship between a percentage of HTLV-III-specific antigen positive (IF-positive) cells (%) and a concentration of DS as was observed in Example11 to be described below,

Figs. 5 and 6 each shows microscopic views of cocultured, Molt-4 cells and Molt-4/HTLV-III cells in the presence of 0 and 10 microgram/ml od DS, respectively, as was obtained in Example 15,

Figs. 7 and 8 each shows a number of viable cells Molt-4/HTLV-III and HTLV-III-specific antigen positive (IF-positive) cells (%) as was observed when the cells were cultured in the presence of AZT and DS, alone and in mixture, respectively, as was observed in Example 17,

Fig. 9 shows a microscopic view of normal Vero cells,

Fig. 10 shows a microscopic view of Vero cells infected with Herpes virus type II,

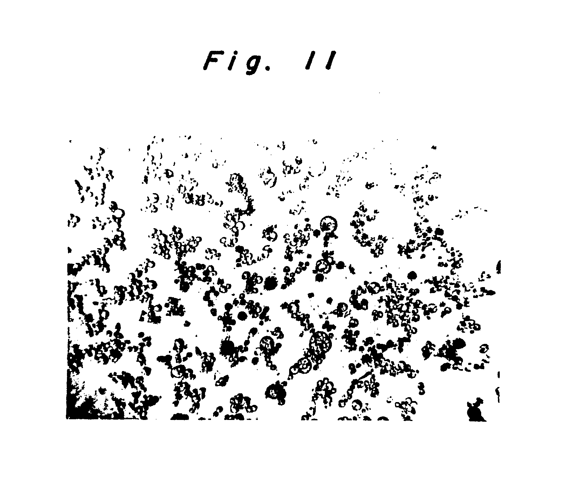

Fig. 11 and 12 each shows microscopic views of Vero cells cultured with 1 $\mu$g/ml or 10 $\mu$g/ml, respectively, of dextran sulfate added simultaneously to adsorption of Herpes virus type II, and

Fig. 13 and 14 each shows microscopic views of Vero cells cultured with 1 $\mu$g/ml or 10 $\mu$g/ml, respectively, of dextran sulfate added after adsorption Herpes virus type II.

DESCRIPTION OF THE PREFERRED EMBODIMENTS

The term "treatment" as used herein is intended to comprehend all the managements of diseases including prevention, sustension (i.e., prevention of aggravation), alleviation (i.e., amelioration of conditions) and therapy.

The virus diseases to be treated according to the present invention include diseases caused by various viruses. Such viruses include Poxviridae (e.g. smallpoxviruse, rabbitpoxvirus, cowpoxvirus, swinepoxvirus, equinepoxvirus, fowlpoxvirus), Herpesviridae (e.g. herpes symplex virus 1 or 2, herpes zoster virus, cytomegalovirus), Adenoviridae, Orthomyxoviridae (e.g. influenza virus), Paramyxoviridae (e.g. parainfluenza virus, mumps virus, respiratory syncytial virus, measles virus), Rhabdoviridae, Picornaviridae (e.g. poliovirus, coxsackie virus, echovirus, hepatitis virus, rhinovirus), Coronaviridae, Parvoviridae, Reoviridae, Togaviridae (e.g. Japenese encephalitis virus, rubella virus), and Retroviridae (e.g. HIVs (e.g. HTLV-III, LAV, ARV etc.), ovine visna virus, equine infectious anemia virus, avian leukemia virus, avian sarcoma virus, avian reticuloendothetiosis virus, murine breast carcinoma virus, murine leukemia virus, murine sarcoma virus, equiline type C virus, hamster type C virus, rat leukemia virus, feline leukemia virus, feline sarcoma virus, feline type Ce virus, ovine leukemia virus, bovine leukemia virus, swine type C virus, simian leukemia virus, Mason-Pfizer Virus, simian sarcoma virus, simian T-lymphotropic virus, baboon type C virus, adult T-cell leukemia virus (ATLV) or human T-lymphotropic virus types I and II (HTLV-I, HTLV-II), human Kawasaki disease virus), etc. The most important diseases are AIDS, PGL (Persistent generalized lymphadenopathy), ARC(AIDS-related complex), LAS(Lymphadenopathy syndrome) and human T-cell leukemia.

As the antiviral agent lacking inhibitory activity against cell-to-cell infection through cell-fusion from virus-infected cell to uninfected cell and/or adsorption of viruses to a target cell which is useful in this invention, there can be empolyed any antiviral agents insofar as they lack inhibitory activity against the above-described cell-to-cell infection, and such antiviral agents include nucleic acid type antiviral agents (for example, 3'-azido-3'-deoxythymidine (azidothymidine or AZT), 2',3'-dideoxynucleosides (2',3'-dideoxyadenosine, -guanosine, -inosine, -cytidine, or -thymidine), ribavirin, isoprinosin, acyclovir, ara A, ara T, ara C, iododeoxyuridine (IDU), bromovinyl deoxyuridine, fluoroiodo aracytosin, 2'-amino-2'-deoxyribofuranosyladenine, trifluridine, acyclovir derivatives (deoxy-, glycyl-or iodo-acyclovir, dihydroxypropoxymethyl guanine, dihydroxybutyl guanine), chloroethyldeoxyuridine, toyocamycin, puromycin, etc.), peptide type antiviral agent (e.g., suramin, distamycin A, actinomycin D, etc.), ansamycin type antiviral agent (e.g., ansamycin, rifamycin, rifampicin, dimethylbenzyl rifampicin, streptovaricin S, etc.), polyanionic type antiviral agents (e.g., HPA-23 (NaSb$_9$ W$_{21}$ O$_{86}$ ), etc.), thiosemicarbatide type antiviral agents (imuthiol, isatin-beta-thiosemicarbazone (IBT), marboran, etc.), phosphoric acid type antiviral agent (e.g., foscarnet), amantadine type antiviral agent (e.g., amantadine, rimantadine, N'-methyladamantane-spiro-3'-pyrolidine hydrochloride, etc.), endogenous antiviral agent (e.g., interferons, interleukins, Neurotropin), glycoside type antiviral agent (e.g., glycyrrhizin, etc.) and lipid type antiviral agent (e.g. AL721 etc.).

Preferred antiviral agents are those having reverse transcriptase inhibitory activity and effective against

HIV (AZT, 2',3'-dideoxynucleosides, suramin, ansamycin, HPA-23, foscarnet), and other agents not having such activity but effective against HIVs (ribavirin, $\alpha$ -, $\beta$ -interferons, AL721, ampligen).

The compound possessing cell-fusion inhibitory activity and/or virus-adsorption inhibitory activity to be used in the present invention includes natural or synthetic oligosaccharides or polysaccharides having at least one S-oxoacid group attached to the saccharic carbon atom through a linking group with a low molecular weight, or pharmaceutically acceptable salts thereof, whichever such oligosaccharides or polysaccharides may be natural or synthetic. The term "natural" means such oligo-or polysaccharides being obtainable by way of such means as extraction from natural resources, such as plants, microbes or animals. The term "synthetic" designates such oligo-or polysaccharides being obtainable by synthetic procedure, for example, by introducing an S-oxoacid group into natural or non-natural (synthetic) oligo-or polysaccarides which have or have not S-oxoacid group.

The term "oligosaccharide" means carbohydrates which contain of 2 to about 9 monosaccarides being each linked with the other.For example,where oligosasccharides contain 3 monosaccharides, for example, one, two or three of such monosaccharides may have at least one S-oxoacid group.

The term "polysaccharide" means carbohydrates which contain of not less than about ten monosaccharides being each linked with the other, where at least one, a minor portion, a major portion or all of the constituent monosaccharides may have at least one but normally not more than four S-oxoacid groups.

The above-described oligosaccharides or polysaccharides may, in place of hydrogen atoms in part of their hydroxyl groups, have short-chain groups such as lower alkyls (e.g. methyl) and lower acyls (e.g. acetyl) or may have sulfate groups linked through such short-chain groups.

The S-oxoacid group includes sulfo group ($-SO_3$ O) and hydroxysulfinyl group ($-SO \cdot OH$), with sulfo group being preferred.

The term "saccharic carbon atom" refers to carbon atoms which constitute a tetrahydrofuran ring or tetrahydropyran ring contained in oligosaccharides or polysaccharides.

The term "low-molecular-weight linking group" is intended to comprehend oxy (-O-), imino (-NH-), thio (-S-), methylene ($-CH_2$ -), ethylidene ($-CH(CH_3$ )-) and the like. The term "low molecular weight" means molecular weights of any linking groups ranging from about 14 to about 32. The preferred linking group includes oxy and imino.

The oligosaccharides or polysaccharides, which are usable in this invention, include natural polysaccarides having at least one hydrogensulfate group ($-O-SO_3$ H) which are obtained from plants or microbes and synthetic polysaccharides having at least one hydrogen sulfate group ($-O-SO$ H) which are produced by esterifying a polysaccharide as obtained from plants or microbes.

Preferred among them are sulfated polysaccharides having non-aminomonosaccharide (containing acids) as a repeating unit, and those containing a trace amount of nitrogen are also included. The monoaminomonosaccharides serving as a repeating unit includes, for example, xylose, arabinose, rhamnose, fucose, glucose, galactose, glucuronic acid, galacturonic acid, mannuronic acid, etc. The naturally occurring polysaccharides include carrageenan (galactan sulfate obtainable from <u>Gigartina stellata</u>, <u>Chondrus crispus</u>, etc.) and fucoidin (polyfucose sulfate obtained from brown marine algae of the genus <u>Laminaria</u>). Said caraageenan includes carrageenans having different sulfate group contents, such as *x*-carrageenan, $\lambda$ -carrageenan and *i*-carrageenan. Examples of the synthetic polysaccharides are those which are obtained by sulfating polysaccharides, such as starch and its partial hydrolysates, dextran produced by the genus <u>Leuconostoc</u> and its partial hydrolysates (having a molecular weight of usually 500 to 2,000,000, normally 2,000 to 300,000, preferably 2,000 to 10,000, most sutiably 3,000 to 8,000), pentosan, glycogen, pectin, cellulose, viscous liquid substances of plant origin (gum arabic, tragacanth, etc.), viscid substances of plant origin (those produced from okra, aloe, <u>Brasenia schreberi</u>, etc.), viscous liquid substances from marine and fresh-water algae (alginic acid, laminarin, etc.), polysaccharides derived from microorganisms (lentinan, pullulan, mannan, xylan, etc.) or synthetic polysaccharides. Among such polysaccharides are included known ones (dextran sulfates; refer to European Patent Publication NO. 0066379) and novel ones. Such novel polysaccharides can be produced by the same procedure as employed for known ones, with an example of such production procedures being described in the following.

Chlorosulfonic acid is added dropwise to pyridine in volume 8 to 10 times that of chlorosulfonic acid, while cooling, and a small amount each of formamide and dextran (in amounts about one-fourth of that of chlorosulfonic acid) are added to the mixture, followed by heating at 55 to 65°C under stirring). After stirring is continued for several hours, the solvent is distilled off, and the residue is purified for example by reprecipitation, dialysis, etc. The term "polysulfate" is intended to refer to compounds obtained through additional sulfuric-acid esterification of the polysaccharides having sulfate groups.

The oligosacharides or polysaccharides, which are useful in this invention, include natural polysaccharides having at least sulfo group ($-SO_3$ H) as may be obtained from animals or synthetic polysaccharides having at least one sulfo group (-SO$_3$ H) as may be produced by sulfuric acid esterification of polysaccharides obtained from animals.

Preferred among them are mucopolysaccharides having aminomonosaccharides (inclusive of N-acylated and sulfated (-O-SO$_3$ H or -NHSO$_3$ H) as a repeating unit which may be furthermore able to contain monosaccharides or acids derived therefrom as another repeating unit. The amino acids or their N-acylated derivatives (preferably N-acetylated ones) serving as a repeating unit include glucosamine, galactosamine and their N-acetylated derivatives and sulfates or partial hydrolysates of the said compounds. As the monosaccharides or acids (preferably hexulonic acid), there may be mentioned, for example, glucose, galactose, gluculonic acid, iduronic acid and the like. The mucopolysaccharides containing such repeating units include, for example, heparin, keratan sulfates, chondroitin-4-sulfate, chondroitin-6-sulfate, dermatan sulfate, teichuronic acid, hyaluronic acid, heparitin sulfate, chitin, chitosan and their partial hydrolysates and modified derivatives (e.g., partially acylated derivatives) and synthetic polysaccharides having such repeating units.

The mucopolysaccharide polysulfates are defined as the compounds which are obtained by additional sulfation of the above mucopolysaccharides. The sulfation can be carried out for example by the procedure as described in Japanese Patent No. 9570/1971, and is generally conducted by reacting polysaccharides with conc. sulfuric acid or its reactive derivatives such as chlorosulfonic acid.

This reaction is carried out normally in the absence or presence of solvent at lowered temperatures. The reaction product is isolated by the conventional procedures, such as neutralization, concentration, precipitation, reprecipitation and chromatography.

The mucolipids and mucoproteins are polysaccharides consisting of mucopolysaccharide and lipid or protein, and their sulfates can be also synthesized by a procedure similar to the above-described one.

The term "pharmaceutically acceptable salt" is intended to designate salts which retain biological activity of the parent compounds but do not exhibit any adverse toxicity at normal dose levels. Such salts include salts with inorganic bases, such as sodium, potassium and aluminium salts, salts with organic bases, such as diethanolamine and amino acid salts. These salts are produced from their corresponding free acids. Such mucopolysaccharides or their sulfates and salts thereof are usable alone and also as a mixture with salts with such metals as zinc and aluminum.

The anti-virus antibody is obtained, as such by administering a viral antigen (e.g., surface antigen) or a fraction containing antigenic determinant to an vertebrate (preferably mammals) to induce an immunoreaction, followed by collection from body fluid of the animal, or in the form of monoclonal antibody by utilizing animal cells.

The above antiviral agent lacking cell-fusion and/or virus-adsorption inhibitory activity and the cell-fusion and/or virus-adsorption inhibitor may be administered concurrently or sequentially. However, both of them should in principle be administered on the same day, optionally at different point of time in the same day but as close to each other as possible. Naturally, these two kinds of active ingredients may be administered simultaneously in the form of a combination of their individual pharmaceutical preparations or they may be contained in the same, single pharmaceutical preparation.

In the present invention, combination of the said antiviral agent and cell-fusion and/or virus-adsorption inhibitor is not limited specifically, but in selecting such combination, adequate consideration may be given to types of the above antiviral agent and cell-fusion and/or virus-adsorption inhibitor depending upon the conditions of patients, kind of diseases, etc. For example, selection is made of a combination of 3'-azido-3'-deoxythymidine and Acylovir as antiviral agent and sodium dextran sulfate as cell-fusion and/or virus-adsorption inhibitor.

The dosage of the antiviral agent may be such an amount as may be sufficient to realize the concentration permitting development of antiviral activity in body, even when it is administered alone. Since the present invention can produce synergism, nevertheless, the antiviral agent may in some instances be given in doses less than the amount producing the above described concentration, for example, 1/2 to 1/200, 1/10 to 1/100 or 1/20 to 1/50 of the said amount. The specific dosage can vary largely depending upon the type and nature of the individual pharmaceutical preparations, and may be changed with the conditions of patients, kind of diseases, and type and amount of the cell-fusion and/or virus-adsorption inhibitor to be combined. For example, Amantadin may be administered at a daily dose of 15 to 150 mg, with Methisazone being given at a daily dose of 20 to 400 mg/kg.

The cell-fusion and/or virus-adsorption inhibitor comprehends any cell-fusion and/or virus-adsorption inhibitors, whether or not they have antiviral activity. The dosage of the cell-fusion and/or virus-adsorption inhibitor having antiviral activity (most of the inhibitors exemplified above fall into the same scope) may be such an amount as may be sufficient to realize the concentration permitting development of antiviral activity in body, even when it is administered solely. Since the present invention can produce antiviral synergism, however, the cell-fusion and/or virus-adsorption inhibitor may in some instances be given in doses less than the amount producing the above-described concentration, for example, 1/2 to 1/500, 1/5 to 1/200 or 1/20 to 1/50 of the said amount. The specific dosage of the cell-fusion and/or virus-adsorption inhibitor may vary depending upon the nature of individual agents, conditions of patients, kind of diseases and type and amount of the antiviral agent to be combined, and generally ranges from 0.02 to 200 mg/kg, preferably 0.1 to 100 mg/kg, with the agent being administered to human being at a daily dose of about 1 mg to 10 g, preferably about 5 mg to 5 g.

Each of the above pharmaceutical preparations may be administered once a day or as divided into two to six times or as a sustained release dosage form.

The administration can be made through any optional routes, such as by oral and topical application and injection.

For administration, the active ingredients may be admixed with a pharmaceutical carrier, such as organic and inorganic solid or liquid excipient, being suitable for such administration route as oral administration, injection, etc. and processed in the form of the conventional pharmaceutical preparations. Such pharmaceutical preparations include solid ones, such as tablets, granules, powders and capsules, and liquid ones, such as solutions, emulsions and suspensions. The above carriers include, for example, starch, lactose, glucose, sucrose, dextrins, celluloses, paraffins, fatty acid glycerides, water, alcohols and gum arabic. If necessary, there may be added adjuvants, stabilizers, emulsifiers, lubricants, binders, pH-regulating agents, isotonic agents and other conventional additives.

Acute toxicities of the antiviral agents to be used in this invention are generally known, while those of the cell-fusion and/or virus adsorption inhibitors are mostly known, and this may be exemplified by the facts that sodium dextran sulfate (having a molecular weight of 7,000 to 8,000 and a sulfur content of 17 to 20 %) exhibits acute toxicities ($LD_{50}$) of 21,000 mg/kg and 4,500 mg/kg, when given orally and intravenously to mice, respectively, while sodium chodroitin sulfate shows acute toxicities ($LD_{50}$) of 7,500 mg/kg and 4,000 mg/kg, when given orally and intraperitoneally to mice, respectively, with no poisoned nor dead case being observed. Heparin sodium and heparin calcium each shows an acute toxicity ($LD_{50}$) of 1,500 to 2,000 mg/kg when injected intravenously to mice.

The following examples will illustrate the present invention in further detail.

The examples are given below to illustrate this invention in more detail, with the test examples being also described to clarify the effects of this invention, wherein unless otherwise specified, the following cell-culturing conditions were employed in the following respective examples;

(a) For culture of HTLV-III/Molt-4 cells (human lymphocytes);

Cell culture was conducted in RPMI 1640 medium supplemented with 10 % of calf bovine serum under 5 % $CO_2$ atmosphere at 37°C, as was the case with Examples 11, 15, 16, 17, 18, 19, 21, 22, and 23.

(b) For culture of HSV/Vero cells (monkey kidney);

Cell culture was carried out in Eagle's MEM medium supplemented with 5 % of calf bovine serum under 5 % $CO_2$ atmosphere at 37°C, as was the case with Example 20.

(c) For culture of Influenza/MDCK cells (dog kidney);

Cell culture was conducted in Eagle's MEM midum supplemented with 10 % of calf bovine serum under 5 % $CO_2$ atmosphere at 37°C, as was the case with Examples 13 and 14.

Preparation 1

Preparation of chondroitin polysulfate from chondroitin sulfate

Chondroitin sulfate (5g) was added to 95 % sulfuric acid (10 ml) cooled to below -25°C with stirring. After addition, the reaction mixture was stirred at the same temperature for 90 minutes. After the end of the period, the reaction solution was gradually poured onto ice (120 g) with stirring. To the resulting solution was gradually added calcium carbonate with well stirring. The precipitates were filtered off, which, then, were washed well with water. To the combined filtrates (240 ml) was added ethanol (60 ml), and the solution was kept to stand overnight at 5 °C to precipitate calcium sulfate. The precipitates were filtered off, and the filtrate was adjusted to pH 10 with sodium carbonate. After addition of acetic acid to make the solution weakly acidic, the solution was concentrated to about 20 ml, then diluted with ethanol (100 ml), and kept to stand overnight at 5 °C. The precipitates in the solution were isolated with centrifugation, washed with ethanol, and with ether, and dried under vaccum to give the white powders of the title compound (S-content: 13%).

Preparation 2

Preparation of keratan polysulfate from keratan sulfate.

Preparation 1 was repreated except that keratan sulfate (100 mg) is used as a starting material and 1 ml in place of 10 ml of 95% sulfuric acid, to give the title compound (S-content: 10%).

(A) Sodium dextran sulfate
(molecular weight: 7,000-8,000, S-content: 17-20%)
        300 mg
Corn starch        45 mg
lactose        300 mg
Magnesium stearate    5 mg

The above ingredients are mixed, granulated, pressed into tablets and enterically coated according to the conventional procedure.

(B) 3'-Azido-3'-deoxythymidine    25 mg
Corn starch       120 mg
Lactose       300 mg
Magnesium stearate    5 mg

The above ingredients are mixed and filled in hard gelatine capsules according to the conventional procedure.

(A) and (B) are combined into a dose form.

Example 2

Sodium dextran sulfate
(molecular weight: 7,000-8,000, S-content: 17-20%)
        120 mg
3'-Azido-3'-deoxythymidine    100 mg
0.9 % saline    q.s. to   10 ml

The above ingredients are mixed and dissolved according to the conventional procedure to form an injectable solution.

Similarly, an injectable solution can be prepared using sodium dextran sulfate having molecular weight of 5,000 and S-content of 13-14% in place of the above sodium dextran sulfate.

Example 3

Sodium heparin    200 units
3'-Azido-3'-deoxythymidine    50 mg
0.9 % Saline    q.s. to 10 ml

8

## Example 4

```
Calcium heparin      500 units
Ara C                10 mg
Procain hydrochloride    10 mg
Water            q.s. to 10 ml
```

## Example 5

```
(A) Sodium keratan polysulfate    100 mg
Lactose              195 mg
Magnesium stearate       5 mg
```
The above ingredients are mixed and filled in hard gelatine capsules according to the conventional procedure.

```
(B) Amantadine      100 mg
Corn starch        195 mg
Lactose          300 mg
```
The above ingredients are mixed, granulated, and pressed into tablets according to the conventional procedure.

## Example 6

```
3'-Azido-3'-deoxythymidine      100 mg
Anti-HTLV-III virus antibody (as IgG)    10 mg
0.9 % Saline              q.s. to 10 ml
```
The above ingredients are mixed and dissolved according to the conventional procedure to form an injectable solution.

## Example 7

```
Sodium dextran sulfate
(molecular weight: 7,000-8,000, S-content: 17-20%)
                  300 mg
3'-Azido-3'-deoxythymidine      30 mg
Corn starch          45 mg
Lactose            270 mg
Magnesium stearate        5 mg
```

The above ingredients are mixed, granulated, pressed into tablets and enterically coated according to the conventional procedure.

## Example 8

```
Sodium dextran sulfate
(molecular weight: 7,000-8,000, S-content: 17-20%)
                  250 mg
2',3'-Dideoxycytidine      5 mg
Corn starch          50 mg
Lactose            100 mg
Magnesium stearate        5 mg
```

The above ingredients are mixed and filled in hard gelatine capsules according to the conventional procedure.

Example 9

Sodium dextran sulfate
(molecular weight: 7,000-8,000, S-content: 17-20%)
                    300 mg
2′,3′-Dideoxyadenine      50 mg
Corn starch              50 mg
Lactose                  100 mg
The above ingredients are mixed, granulated, pressed into tablets and enterically coated according to the conventional procedure.

Example 10

Sodium dextran sulfate
(molecular weight: 7,000-8,000, S-content: 17-20%)
                    250 mg
3′-Azido-3′-deoxythymidine      20 mg
Acyclovir              30 mg
Lactose                50 mg
Magnesium stearate     10 mg

The above ingredients are mixed, granulated and filled in hard gelatine capsules according to the conventional procedure.

Example 11 (Inhibition of viral infection, HTLV-III)

AIDS virus HTLV-III was challenged to established human T-lymphocyte culture cells MT-4 and the cell suspension and virus particles were incubated at 37°C for 1 hour. The cells were then washed with phosphate buffered saline once and cultured with or without various doses of the test substances in RPMI-1640 medium at 37°C under 5 % $CO_2$ in an incubator.

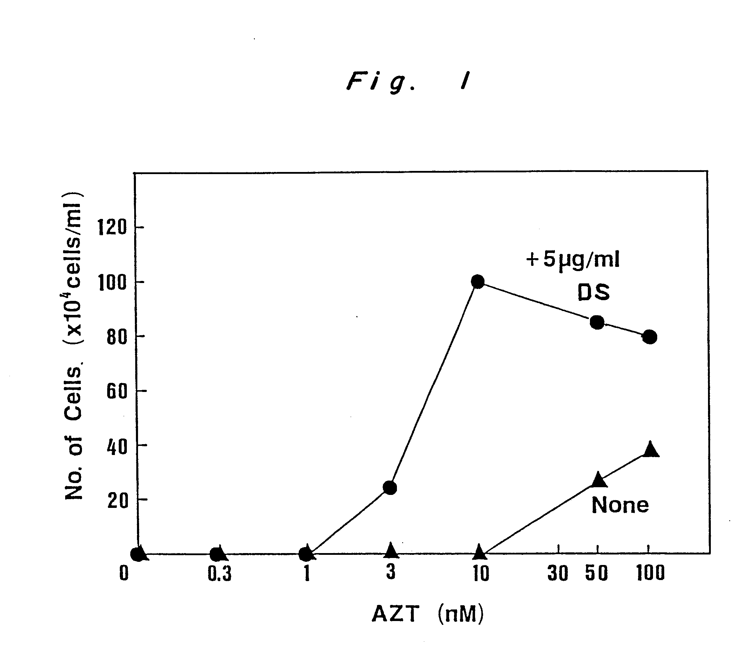

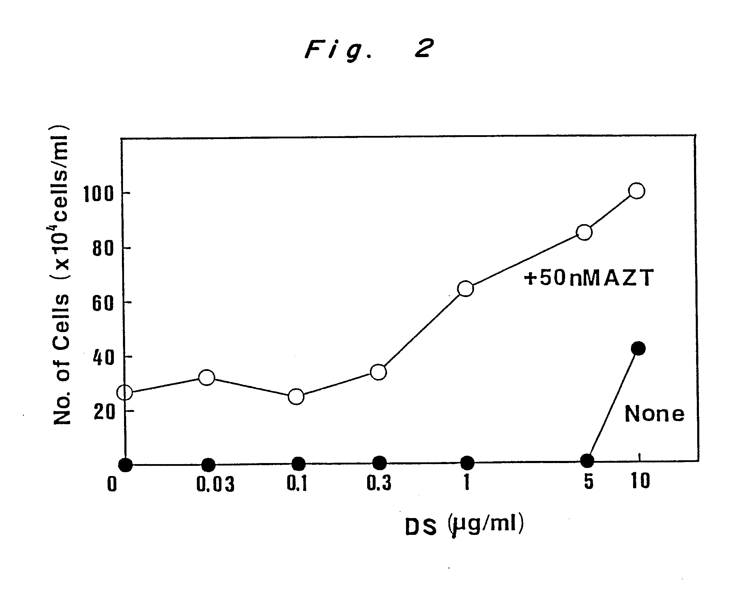

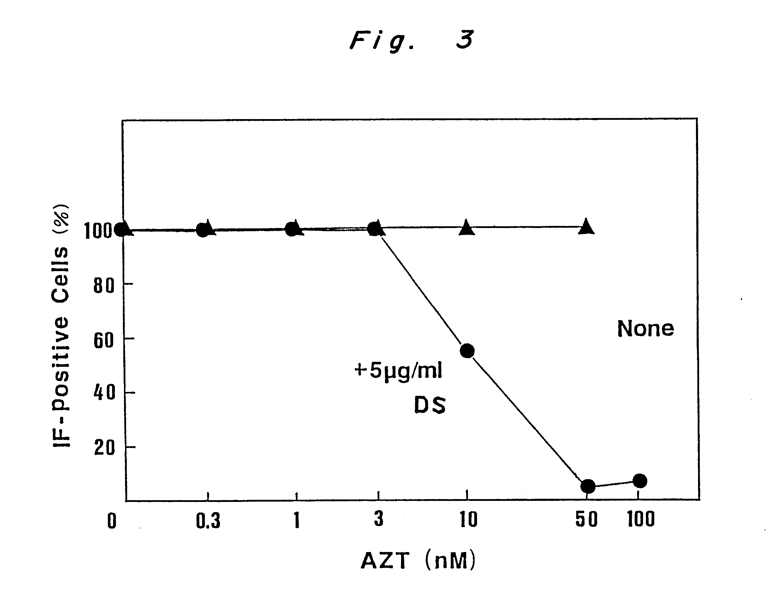

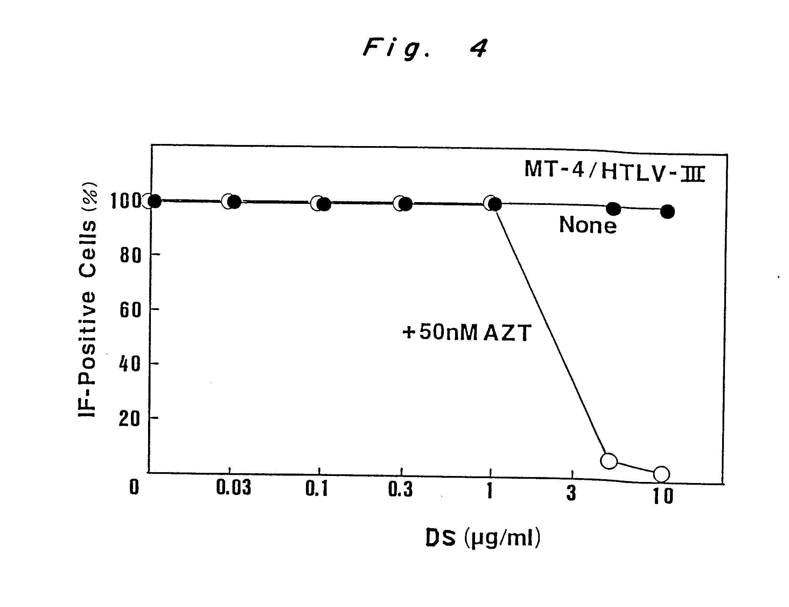

MT-4 cells infected with HTLV-III were destructed as the virus proliferated, resulting in decrease of viable cells. Prior to the cytolysis by viral cytopathic effect, the cells infected with HTLV-III which were detectable by indirct immuno fluorescence method appeared. On day 12, number of the living cells was counted to know the extent of infection, and rate of the infected cells was determined by indirect immuno-fluorescence method using an antibody having specificity to the virus specific antigen. As the test substances, 3′-azido-3′-deoxythymidine (AZT) and dextran sulfate(M.W. 7000 - 8000, S-content 17 - 20%) (DS) were used. The results are shown in Figs. 1 to 4.

It is evident from these data that, by the combined use fo the above two substances, increase in the number of viable cells or decrease in the number of infected cells, i.e., inhibition of infection with and replication of AIDS virus, could be realized even at the concentrations at which not effect could be obtained by the sole use of any of these substances.

Example 12 (Inhibition of viral infection with Herpes Simplex virus)

A suspension of Vero cells was distributed in 1 ml portions into culture test tubes, and the tubes were placed on a slant test tube stand, followed by incubation overnight at 37°C. One arbitrary test tube was taken out to count numbers of cells contained therein. From such number of cells, the concentration (PFU/ml) of a virus suspension to be inoculated was calculated by the following equation:
Multiplication of infection (moi) = V/C (= 0.2)
where; V is a number of viruses
C is a number of cells
A stock solution of HSV Type II (SAV) viruses was diluted to a specifically determined virus concentration using the culture solution containing 450 ml of MEM (MEM prepared by dissolving 9.4 g of Eagle MEM Medium No. 1 in 100 ml of redistilled water), 5 ml of a 2.66 % glutamine solution, 25 ml of calf bovine serum and 7.5 ml of a 7.0 % $NaHCO_3$ solution, and the thus diluted stock solution was inoculated in 0.1

10

ml portions onto the culture test tubes. After infection was allowed at 37°C for 1 hour, 0.9 ml of the culture solution was added to the test tubes, and incubation was carried out at 37°C for 20 hours, followed by evaluation oc cytopathic effect (CPE) caused.

With various antiviral agents and drugs (sulfated polysaccharides) being picked up as test substances, the above test procedures were conducted in the following three modified manners: namely, (1) the first culture solution for dilution of the SAV stock solution and the second culture solution for incubation both were exployed without addition of any test substances to run control test, (2) both the first and second culture solutions were incorporated with test substance(s) to carry out tests on virus-adsorption inhibitory activity (Method 1), and (3) the first culture solution was used without addition of test substances(s), with the second one being admixed with test substance(s), to conduct tests on cell-fusion inhibitory activity (Method 2).

The cytopathic effect (CPE) as evaluated was expressed by the following rating symbols:

+ + : Nearly the same as in the case of control (100 % infection)

+ : 10 to 40 % of cytopathic effect observed

-: Above 40 % of cytopathic effect observed

### Plague assay

After evaluation of cytophathic effect, the test tubes were subjected to three refrigeration (dry ice/acetone)-thawing cycles, and respective test specimens were diluted with Hank's solution in step-wise to 10 to 100,000 times and each distributed in equal portions into two Petri dishes (35 mm diameter) containing 2.5 ml of Vero cell suspension, followed by incubation. Upon unilayer formation of cell growth, the culture broths were almost removed by suction, and the diluted SAV stock solution was inoculated in 0.1 ml/dish to the Petri dishes. After infection through adsorption at 37°C for 1 hours, the dishes were overlaid with about 2.5 ml of the above culture solution admixed with 2 % methylcellulose, subjected to incubation at 37°C for 3 days and incorporated with 1.5 ml of 0.015 % Neutral Red solution, followed by culture overnight at 37°C to count the numbers of plaques formed. Based on the numbers of plaques, the virus yeilds (PFU/ml) were determined, with the relative viral replication supressing activities of treated specimens against controls being tabulated in the following tables.

Calculation;

$$\text{Inhibition Value}\left(\frac{PFU}{PFU}\right) = \frac{\text{Yield of virus (Control)}}{\text{Yield of virus (Treated)}}$$

As the test substances, the following compounds were used:-

alginic acid sulfate     S = 14%

chondroitin-4-sulfate polysulfate(A)     S = 6%

chondroitin-4-sulfate polysulfate(B)     S = 16%

chondroitin-6-sulfate polysulfate(A)     S = 8%

chondroitin-6-sulfate polysulfate(B)     S = 15%

chitin sulfate     S = 9%

chitosan sulfate     S = 18%

Dextran sulfate, molecular weight 5,000(DS-S) S = 14%

Dextran sulfate, molecular weight 500,000(DS-L) S = 14%

(Note) S: S-content (%)

Inhibition values for each combination of test agents are shown in the Table below.

Method 1

| Antiviral Agent(μg/ml) | | Ara C (μg/ml) | | |
|---|---|---|---|---|
| Compound | (μg/ml) | 0 | 0.5 | 1.0 |
| | 0 | (1) | 9 | 68 |
| DS-S | 0.5 | 1 | 13 | 94 |
| DS-S | 1.0 | 1 | 38 | 309 |
| DS-L | 0.5 | 1 | 18 | 850 |

Method 1

| Antiviral Agent(μg/ml) | | Amantadine | | | |
|---|---|---|---|---|---|
| Compound | (μg/ml) | 0 | 10 | 50 | 100 |
| | 0 | (1) | 1 | 6 | 8 |
| DS-S | 1 | 2 | 4 | 16 | 78 |

Method 1

| Antiviral Agent(μg/ml) | | Ara A | | |
|---|---|---|---|---|
| Compound | (μg/ml) | 0 | 1 | 5 |
| | 0 | (1) | 1 | 180 |
| DS-S | 1 | 2 | 3 | 450 |

Method 1

| Antiviral Agent(μg/ml) | | Acyclovir | | |
|---|---|---|---|---|
| Compound | (μg/ml) | 0 | 1 | 5 |
| | 0 | (1) | 180 | 900 |
| DS-S | 1 | 2 | 900 | 3000 |

Method 1

| Compound | Antiviral Agent (µg/ml) | | IDU | | |
|---|---|---|---|---|---|
|  | (µg/ml) | 0 | 1 | 5 | 10 |
|  | 0 | (1) | 1 | 110 | 110 |
| DS-S | 1 | 1 | 2 | 275 | 440 |
| DS-L | 1 | 1 | 2 | 440 | 1100 |

Method 2

| Compound | Antiviral Agent (µg/ml) | | Ara C | | |
|---|---|---|---|---|---|
|  | (µg/ml) | 0 | 0.1 | 0.5 | 1.0 |
| Dextran sulfate | 0 | (1) | 2 | 4 | 14 |
|  | 1 | 1 | 4 | 17 | 56 |
|  | 10 | 3 | 9 | 17 | 79 |

Method 2

| Compound | Antiviral Agent (µg/ml) | Ara T | | |
|---|---|---|---|---|
|  | (µg/ml) | 0 | 5 | 10 |
|  | 0 | (1) | 13 | 23 |
| DS-S | 10 | 2 | 35 | 56 |

Method 1

| Compound | Antiviral Agent (µg/ml) | Acyclovir | |
|---|---|---|---|
|  | (µg/ml) | 0 | 5 |
|  | 0 | (1) | 180 |
| chondroitin-4-sulfate polysulfate (A) | 1 | 1 | 514 |
| chondroitin-4-sulfate polysulfate (B) | 1 | 1 | 857 |

Method 1

| Antiviral Agent (µg/ml) | | Acyclovir | |
|---|---|---|---|
| Compound | (µg/ml) | 0 | 1 |
| | 0 | (1) | 53 |
| chondroitin 6-sulfate | 1 | 1 | 160 |
| polysulfate (A) | | | |
| chondroitin-6-sulfate | 1 | 1 | 80 |
| polysulfate (B) | | | |

Method 1

| Antiviral Agent (µg/ml) | | Acyclovir | |
|---|---|---|---|
| Compound | (µg/ml) | 0 | 1 |
| | 0 | (1) | 51 |
| chitosan sulfate | 1 | 1 | 180 |

Method 1

Virus yield (untreated/treated)

| Antiviral Agent (µg/ml) | | Ara C | | |
|---|---|---|---|---|
| Compound | (µg/ml) | 0 | 0.5 | 1.0 |
| | 0 | (1) | 500 | 1333 |
| alginic acid sulfate | 1 | 1 | 2667 | 4444 |
| chondroitin-4-sulfate | 1 | 2 | 20000 | 20000 |
| polysulfate (A) | | | | |
| chondroitin-4-sulfate | 1 | 3 | 8000 | 13333 |
| polysulfate (B) | | | | |
| chondroitin-6-sulfate | 1 | 3 | 2500 | 5000 |
| polysulfate (A) | | | | |
| chondroitin-6-sulfate | 1 | 1 | 8000 | 40000 |
| polysulfate (B) | | | | |

| | | | | |
|---|---|---|---|---|
| chitin sulfate | 100 | 3 | 5000 | 4000 |
| chitosan sulfate | 1 | 1 | 1250 | 4000 |

It is evident from the above results that the antiviral effect could be significantly enhanced by combining amantadine, acyclovir, ara A, ara C, ara T and IDU, which are anti-virus agents having no cell fusion inhibitory activity, with alginic acid sulfate, chondroitin sulfate polysulfates, chitin sulfate, chitosan sulfate, or dextran sulfate.

Example 13 (Inhibition of viral infection, influenza virus)

MDCK cells derived from dog kidney were allowed to proliferate sufficiently in a culture test tube (to form a mon layer), and washed once with phosphate buffered saline(PBS(-)). Type-A influenza virus was diluted with a culture medium to $1.0 \times 10^6$ PFU/ml and 0.2 ml of the obtained suspension was inoculated onto the cells to allow infection. In an experiment using dextran sulfate with a molecular weight of 5000(S-content 14%) (DS-S) or 500000(S-content 14%) (DS-L) together with amantadine, these substances at various concentrations shown in the table below were added to the virus suspension immmediately before the use for infection. The mixture was allowed to stand at room temperatrure for 30 minutes to allow infection of virus, followed by addition of a culture medium (1 ml) containing the said substances at the concentrations shown below. After incubation at 37°C for 24 hours, judgements were made in such manner that when the rate of the formed CPE (Cytopathic effect) was 0 - 10 %, the result was + +, when it was 10 - 40 %, the result was +, and when it was greater than 40 %, the result was -. (There was found more than 80 % CPE formed in the culture test tube without the test substances.) The results are shown in the following table.

| | Antiviral Agent (µg/ml) | Amantadine | | | | |
|---|---|---|---|---|---|---|
| Compound (µg/ml) | | 0 | 0.1 | 1 | 10 | 100 |
| | 0 | - | - | + | ++ | ++ |
| DS-S | 1 | - | - | + | ++ | ++ |
| " | 10 | - | - | + | ++ | ++ |
| " | 100 | - | + | ++ | ++ | ++ |
| " | 1000 | - | + | ++ | ++ | ++ |

| | Antiviral Agent (µg/ml) | Amantadine | | | | |
|---|---|---|---|---|---|---|
| Compound (µg/ml) | | 0 | 0.01 | 1 | 10 | 100 |
| | 0 | - | - | - | + | + |
| DS-L | 0.1 | - | - | - | + | + |
| " | 1 | - | - | + | + | ++ |

It is evident from the above results that the antiviral effect was synergistically improved by the combined use of amantadine and dextran sulfate.

Example 14 (Inhibition of viral infection)

MDCK cells derived from dog kidney were sufficiently proliferated (to form a mono layer) in a 96-well microplate, and washed once with phosphate buffered saline (PBS(-)). Type-A influenza virus was diluted with culture medium to 1.0 X $10^6$ PFU/ml, and 0.025 ml of the obtained suspension was inoculated onto the cells to allow infection. The substances were added in the same amount as shown in the table below to the virus suspension at the specified concentrations immediately before the use for infection. The mixture was allowed to stand at room temperature for 30 minutes to allow infection of virus, followed by addition of a culture medium (100 μl) containing the test substances at the concentrations shown below. After incubation at 37°C under 5 % $CO_2$ for 44 hours, judgements were made in the same manner as Example 9. The results are shown in the following table.

| Antiviral Agent (μg/ml) | | Amantadine | | | | |
|---|---|---|---|---|---|---|
| Compound (μg/ml) | | 0 | 0.1 | 1 | 10 | 100 |
| | 0 | − | − | −− | + | + |
| alginic acid sulfate | 1 | − | − | − | + | + |
| (Sulfur content=14%) | | | | | | |
| " | 10 | − | + | + | + | ++ |
| " | 100 | ++ | ++ | ++ | ++ | ++ |

| Antiviral Agent (μg/ml) | | Amantadin | | | | |
|---|---|---|---|---|---|---|
| Compound (μg/ml) | | 0 | 0.1 | 1 | 10 | 100 |
| | 0 | − | − | − | − | + |
| chondroitin-4-sulfate | 1 | − | − | − | − | + |
| (Sulfur content=16%) | | | | | | |
| " | 10 | − | − | − | − | + |
| " | 100 | − | − | − | + | + |
| " | 1000 | − | − | − | + | + |

It is evident from the above results that the antiviral effect was synergistically improved by the combined use of amantadine and alginic acid sulfate or chondroitin sulfate polysulfates.


Example 15 (Inhibition of cell fusion)

By means of a cell to cell infection system using established human T-cell Molt-4 (OKT $4^+$ ) and its AIDS virus producing cell line Molt-4/HTLV-III, activity of dextran sulfate having a molecular weight of 7,000 - 8,000 and a sulfur content of 17 -20 % (DS) against the cell to cell infection was evaluated. The cultivation was conducted as follows:-

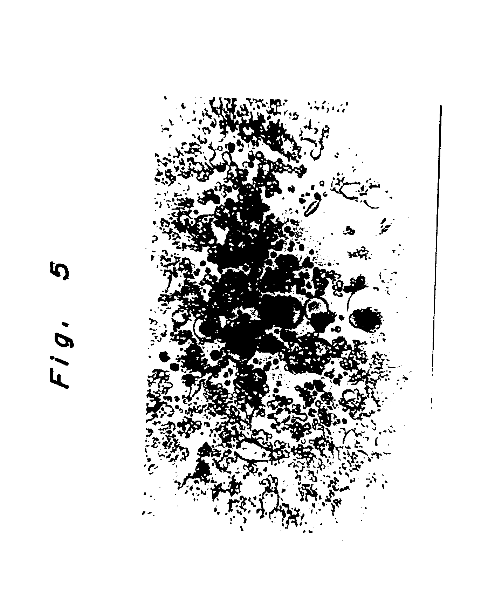

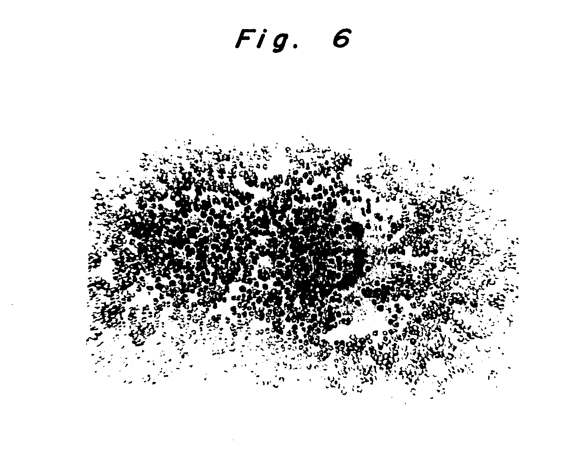

Molt-4 cells (uninfected) and Molt-4/HTLV-III cells (virus productive) were mixed in a cell ratio of 9 : 1 and incubated in an RPMI medium with or without dextran sulfate. In this system, the Molt-4/HTLV-III cells successively fuse with Molt-4 cells to form giant cells and then perish. When the Molt-4 cells and the Molt-4/HTLV-III cells were concurrently cultured without dextran sulfate, giant cells infected through cell fusion appeared (about 30 cells in the visual field) as shown in Fig.5. When the concurrent culture was carried out by adding 10 μg/ml of DS, no giant cell was observed as shown in Fig. 6. This was indicative of the fact that the dextran sulfate inhibited the cell fusion between the cells infected with virus and the cells not infected with virus. In other words, the dextran sulfate inhibited the direct cell-to-cell infection of AIDS virus.

Example 16 (Suppression of Virus release ammount)

HTLV-III producing Molt-4/HTLV-III cells (all being positive to antigen having specificity to virus) and the uninfected Molt-4 cells were mixed at a cell ratio of 1:9, and the mixture was incubated in a culture medium containing 10, 50, and 100 $\mu$g/ml of dextran sulfate (molecular weight 7,000 - 8,000, sulfur content 17 - 18 %) (DS). The culture medium were renewed every three days. On day 9, number of the living cells was counted, and the virus release amount was determined by reverse transcriptase activity (the viruses released into the medium were separated by centrifugation and diluted to remove possible inhibitory effect of DS which may be present in the remaining medium). Also, the rates of cells positive to virus antigen (infectred cells) were determined by an indirect immunofluororescence method. The results are shown in the following table.

| DS ($\mu$g/ml) | 0 | 10 | 50 | 100 |
|---|---|---|---|---|
| Virus release amount (RT activity) ($\times 10^4$ cpm/ml) | 405 | 162 | 95 | 78 |
| Rate of infected cells (%) | 100 | 100 | 27 | 21 |

It is evident from the above results that the virus release amount significantly decreased and the non-infected cells still remain in the case of DS containing medium, that is to say, that the dextran sulfate inhibited the release of virus from the infected cells.

Example 17 (Inhibition of viral adsorption)

Procedure

Virions from HTLV-III were added to a culture medium containing 10$\mu$g/ml of test compounds, which was immediately added to Mt-4 cells. After incubating at 37°C for 60 minutes, the culture was cetrifuged and the supernatant was removed. Viruses which were not adsorbed to the cells were removed by washing twice with physiological phosphate buffer. Fresh culture medium was added to the washed cells and the culture was incubated at 37°C under 5% $CO_2$ for 3 days. On day 3, the effects were compared by determining the rate of infected cells (%) by indirect fluorescent antibody technique.

## Results

| Compound | M.W. | S-content* | Rate of infected cells** (%) |
|---|---|---|---|
| Non (uninfected control) | – | .– | 0 |
| Non (infected control) | – | – | 100 |
| λ-carrageenan | – | ≅16 | <5 |
| alginic acid sulfate | 50,000 ∿300,000 | ≅14 | <5 |
| chitosan sulfate | – | 18 | <5 |
| dextran sulfate | 500,000 | 16 | <5 |
| dextran sulfate | 7,000 ∿8,000 | 17∿20 | <5 |
| chondroitin polysulfate | 5,000 ∿8,000 | 13 | <5 |

\* S-content is originated from sulfate and sulfonate groups attached to the saccharic chain.

\*\* Rate of infected cells=fluorescent positive cells×100/total cells

From the above results, it can be clearly seen that the test compounds, dextran sulfate and other similar sulfated polysaccharide strongly inhibited the process of adsorption of HIV virion to the receptor of target cell, i.e. the first step of the establishment of viral infection.

Consequently, it can be inferred that the cell fusion inhibiting activity of dextran sulfate and other similar sulfated polysaccharide was resulted from an inhibiting activity against the bonding of HIV antigen presumably generated on the surface of the infected cell to the receptor of the uninfected cell.

### Example 18 (Inhibition of viral infection)

### Procedure

In the same procedure as in Expample 7, the MT-4 cells infected with HTLV-III were cultured in medium and the changes in the number of viable cells and the rate of the infected cells were recorded in time course. Four kinds of culture mediums were used, i.e., without addition, with 10 nM.AZT, with 5 μg/ml dextran sulfate, and co-existence of 10 nM.AZT and 5 μg/ml dextran sulfate(M.W. 7000 - 8000, S-content 17 - 20%).

Results

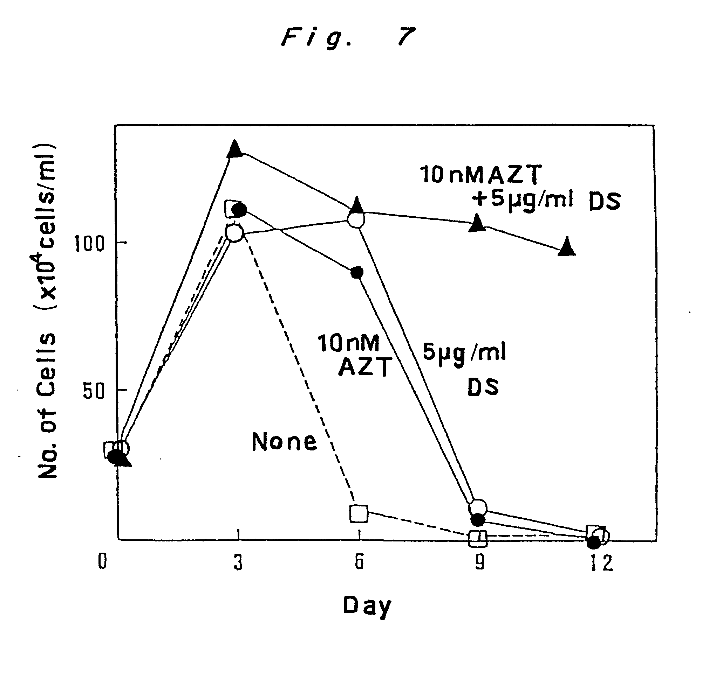

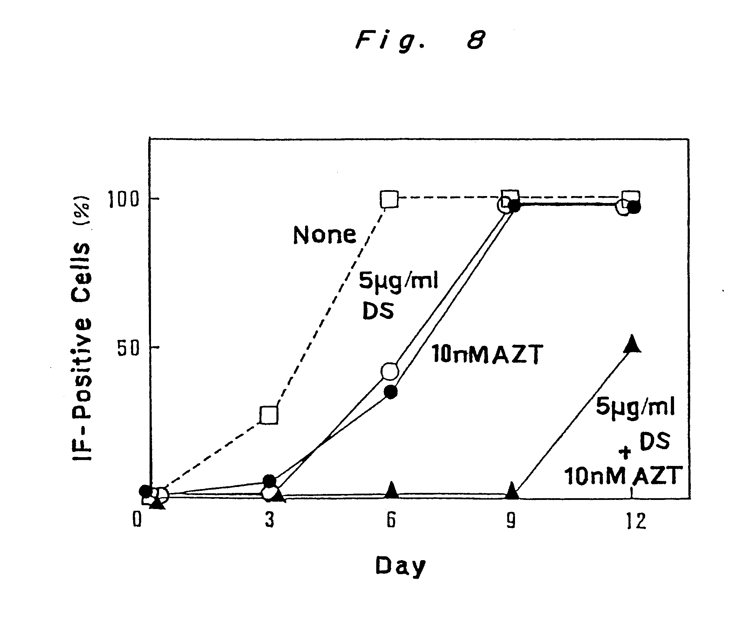

As shown in Fig. 7 (number of viable cells) and Fig. 8 (rate of infected cells), in the case of the sole use of 10 nM.AZT or 5 μg/ml dextran sulfate, complete infection was slightly retarded in comparison with the case of non-addition, but 100 % infection was attained as in the case of non-addition on day 9. However, in the case of combined use of 10 nM.AZT and 5 μg/ml dextran sulfate, no change was seen in the number of viable cells as compared with the case of non-infection up to day 12, and rate of infected cell was also defenately low.

Example 19 (Inhibition of viral infection)

Procedure

In the same procedure as in Example 7, the MT-4 cells infected with HTLV-III were cultured in medium containing dextran sulfate (M.W. 7000 - 8000, S-content 17 - 20%) (DS) and antiviral agents (2',3'-dideoxythymidine and 2',3'-dideoxyadenosine) in various concentration. On day 12, rate of infected cells (%) was determined by an indirect immunofluorescence method in a manner similar to that in Example 7.

Results

In the following Tables, values indicate the rate of infected cells.

1) Combination of 2',3'-deoxythymidine and DS

| Antiviral Agent (nM) | 2',3'-deoxythymidine | | |
|---|---|---|---|
| Compound | 0 | 10 | 50 |
| DS  0μg/ml | 100 | 100 | 100 |
| 5μg/ml | 100 | 50 | 5 |

2) Combination of 2',3'-deoxyadenosine and DS

| Antiviral Agent (nM) | 2',3'-deoxyadenosine | | | |
|---|---|---|---|---|
| Compound | 0 | 50 | 200 | 1000 |
| DS  0μg/ml | 100 | 100 | 100 | 100 |
| 5μg/ml | 100 | 90 | 45 | 5 |

3) Combination of suramin and DS

| Antiviral Agent | suramin | | |
|---|---|---|---|
| Compound | 0μg/ml | 0.1μg/ml | 0.6μg/ml |
| DS  0μg/ml | 100 | 100 | 100 |
| 5μg/ml | 100 | 100 | 20 |

4) Combination of HPA-23 and DS

| Antiviral Agent | HPA-23 | | |
|---|---|---|---|
| Compound | 0μg/ml | 10μg/ml | 40μg/ml |
| DS  0μg/ml | 100 | 100 | 100 |
| 5μg/ml | 100 | 80 | 20 |

From the above results, it can be clearly seen that the effect of DS as a synergist is exerted not only in the combination with 3'-azido-3'-deoxythymidine but also with other anti-AIDS antiviral agents.

Example 20 (Inhibition of cell fusion)

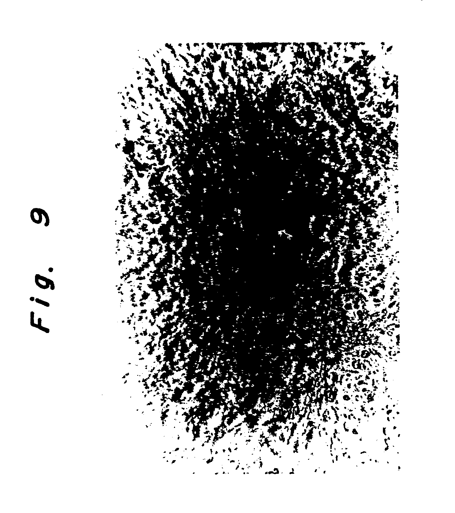

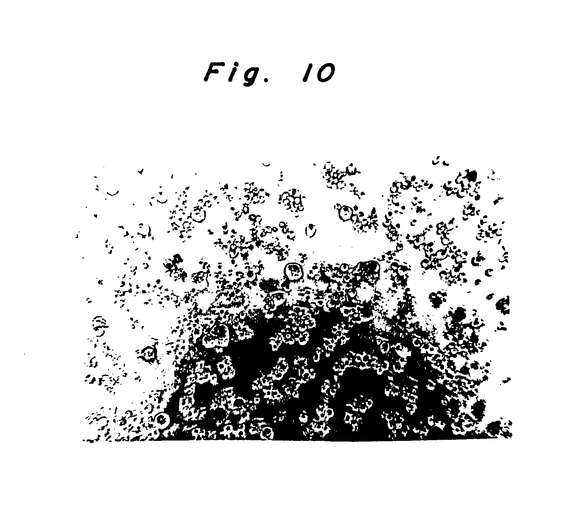

Fusion of Vero cells derived from African green monkey kidney can be induced when monolayer of said cells are infected with Herpes virus type II (Fig. 9, 10). Activity of dextran sulfate (M.W. 5,000) (DS-S) against cell fusion inducible by viral infection with Herpes virus was determined using the above infection system.

In a culture system in which DS-S was added simultaneously with viral adsorption on Vero cells, cytopathic effect due to viral infection as well as incidence of cell fusion were observed, as shown in Fig.

11, in the presence of 1 µg/ml DS-S.

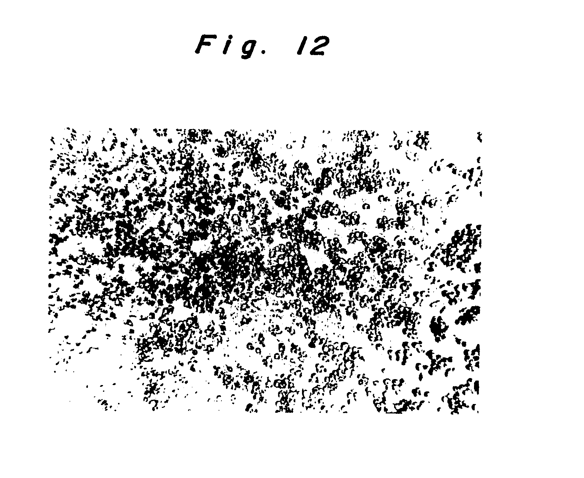

With 10 µg/ml dextran sulfate, however, no cell infection was observed as shown in Fig. 12.

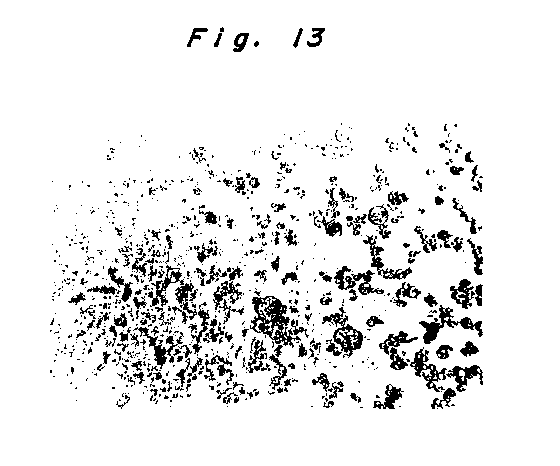

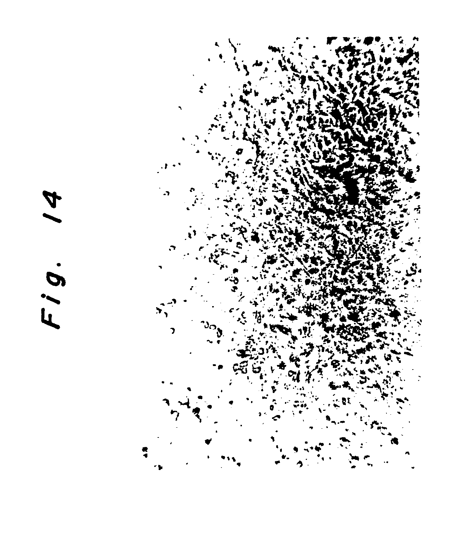

In another system in which dextran sulfate was added after one hour from the viral adsorption on Vero cells, also cytopathic effect due to viral infection as well as incidence of cell fusion were observed, as shown in Fig. 13, in the presence of 1 µg/ml dextran sulfate. With 10 µg/ml dextran sulfate, however, incidence of cell fusion was not observed while cytopathic effect due to viral infection appeared (Fig. 14).

These results indicate that the activity of dextran sulfate inhibiting viral infection of Herpes virus includes a step inhibiting cell fusion alone between steps of no inhibition and of complete inhibition depending on condition of application and/or concentration of dextran sulfate. Thus, dextran sulfate has an activity inhibiting cell fusion in the course of infection of cells with Herpes virus.

Example 21 (Inhibition of viral infection)

Procedure

MT-d cells, infected with HTLV-III according to Example 7, were cultured in the medium with or without various doses of cell fusion inhibitors (condroitin-4-sulfate polysulfate (S-content 16%) and chitosan sulfate-(S-content 18%) and antiviral agents (3'-azido-3'-deoxythymidine and 2',3'-dideoxycytidine). On day 12, rate of infected cells (%) was determined by the fluorescent antibody method as in Example 7.

Results

1) Use of 3'-azido-3'-deoxythymidine and condroitin-4-sulfate polysulfate

| condroitin-4-sulfate polysulfate \ 3'-azido-3'-deoxythymidine | 0nM | 5nM | 10nM |
|---|---|---|---|
| 0 µg/ml | 100 | 100 | 100 |
| 5 µg/ml | 100 | 50 | 5 |

2) Use of 2',3'-dideoxycytidine and condroitin-4-sulfate polysulfate

| condroitin-4-sulfate polysulfate \ 2',3'-dideoxycytidine | 0nM | 10nM | 50nM |
|---|---|---|---|
| 0 µg/ml | 100 | 100 | 100 |
| 5 µg/ml | 100 | 60 | 10 |

3) Use of 3'-azido-3'-deoxythymidine and chitosan sulfate

| 3'-azido-3'-deoxythymidine chitosan sulfate | 0nM | 10nM | 50nM |
|---|---|---|---|
| 0 µg/ml | 100 | 100 | 100 |
| 5 µg/ml | 100 | 50 | 7 |

4) Use of 2',3'-dideoxycytidine and chitosan sulfate

| 2',3'-dideoxycytidine chitosan sulfate | 0nM | 1nM | 10nM |
|---|---|---|---|
| 0 µg/ml | 100 | 100 | 100 |
| 5 µg/ml | 100 | 55 | 10 |

Example 22 (Inhibition of viral infection)

Procedure

MT-4 cells, infected with HTLV-III according to Example 7, were cultured in the medium with or without various doses of dextran sulfate(MW:7000-8000, S = 17-20%) and antiviral agents (acyclovir, glycyrrhizin and neurotropin). On day 12, rate of infected cells (%) was determined by the fluorescent antibody method as in Example 7.

Results

| acyclovir dextran sodium sulfate | 0µM | 40µM | 80µM |
|---|---|---|---|
| 0 µg/ml | 100 | 100 | 100 |
| 5 µg/ml | 100 | 20 | 5 |

| glycyrrhizin<br>dextran sodium sulfate | 0μg/ml | 100μg/ml | 200μg/ml |
|---|---|---|---|
| 0 μg/ml | 100 | 100 | 100 |
| 5 μg/ml | 100 | 80 | 60 |

| neurotropin<br>dextran sodium sulfate | 0μg/ml | 100μg/ml | 200μg/ml |
|---|---|---|---|
| 0 μg/ml | 100 | 100 | 100 |
| 5 μg/ml | 100 | 70 | 55 |

Example 23 (Inhibition of viral infection)

Procedure

MT-4 cells, infected with HTLV-III according to Example 7, were cultured in the medium with or without various doses of cell fusion inhibitors and antiviral agents as shown in the following Tables. On day 12, rate of infected cells (%) was determined by the fluorescent antibody method as in Example 7.

Results

| Antiviral Agent Compound | 3'-azido-3'-deoxythimidine 0nM | 10nM | 50nM | 2',3'-dideoxycytidine 0nM | 1nM | 10nM | acyclovir 0μM | 40μM | 80μM |
|---|---|---|---|---|---|---|---|---|---|
| γ-carrageenan (S=12%) 0 μg/ml | 100 | 100 | 100 | 100 | 100 | 100 | 100 | 90 | 90 |
| 5 μg/ml | 100 | 30 | 5 | 100 | 70 | 10 | 100 | 70 | 50 |
| fucoidin (S=15%) 5 μg/ml | 100 | 50 | 5 | 100 | 30 | 10 | 100 | 70 | 40 |
| algic acid sulfate (S=19%) 5 μg/ml | 100 | 20 | 3 | 100 | 60 | 40 | 100 | 80 | 40 |
| chitin sulfate 50μg/ml (S=9%) | 100 | 80 | 50 | 100 | 80 | 70 | 100 | 70 | 70 |
| heparin (S=13%) 10μg/ml | 100 | 50 | 10 | 100 | 50 | 10 | 100 | 60 | 40 |
| keratan sulfate (S=10%) 10μg/ml | 100 | 80 | 60 | 100 | 60 | 50 | 100 | 30 | 60 |
| hyaluronic acid sulfate (S=8%) 50μg/ml | 100 | 70 | 50 | 100 | 80 | 80 | 100 | 80 | 80 |
| condroitin polysulfate (S=13%) 5 μg/ml | 100 | 40 | 5 | 100 | 30 | 5 | 100 | 80 | 60 |

## Claims

1. A pharmaceutical composition for treating a disease caused by virus comprising
(A) an antiviral agent lacking inhibitory activity (1) against cell-to-cell infection through cell fusion from a virus infected cell to an uninfected cell and/or (2) against adsorption of a virus to a target cell, and

24

(B) a compound possessing cell fusion inhibitory activity and/or virus-adsorption inhibitory activity, said antiviral agent and compound being administered in respective amounts so that the combined dosage thereof is effective to treat said disease.

2. The composition according to claim 1, wherein the said antiviral agent is an antiviral agent lacking inhibitory activity against cell-to-cell infection through cell fusion from a virus-infected cell to an uninfected cell.

3. The composition according to claim 2, wherein the said antiviral agent is combined with a cell-fusion inhibitor.

4. The composition according to claim 2, wherein the said antiviral agent is combined with a compound serving the dual purposes as a cell-fusion inhibitor and a virus-adsorption inhibitor.

5. The composition according to claim 1, wherein the said antiviral agent is an antiviral agent lacking inhibitory activity against adsorption of a virus to a target cell.

6. The composition according to claim 5, wherein the said antiviral agent is combined with a virus-adsorption inhibitor.

7. The composition according to claim 5, wherein the said antiviral agent is combined with compound serving the dual purposes as a cell-fusion inhibitor and a virus-adsorption inhibitor.

8. The composition according to claim 1, wherein the said antiviral agent is an antiviral agent lacking inhibitory activities both against cell-to-cell infection through cell fusion from a virus-infected cell to an uninfected cell and against adsorption of a virus to a target cell.

9. The composition according to claim 8, wherein the said antiviral agent is combined with a cell-fusion inhibitor or virus-adsorption inhibitor.

10. The composition according to claim 8, wherein the said antiviral agent is combined with compound serving the dual purposes as a cell-fusion inhibitor and a virus-adsorption inhibitor.

11. The composition according to claim 1, wherein said antiviral agent and said compound are present in respective amounts which are individually effective to treat said disease.

12. The composition according to claim 1, wherein the antiviral agent and said compound are present in respective amounts, one of which is less than the dosage thereof which would be effective to treat said disease.

13. The composition according to claim 1, wherein said antiviral agent and said compound are present in respective amounts whcih provide a synergistic effect in the treatment of a disease caused by a virus.

14. The composition according to claim 1, wherein the said antiviral agent is present in an amount lower than its effective dose by iteself against virus.

15. The composition according to claim 1, wherein the said cell-fusion inhibitor and/or virus-adsorption inhibitor lack(s) antiviral activity.

16. The composition according to claim 13, wherein the said cell-fusion inhibitor and/or a virus-adsorption inhibitor and are (is) present in an amount lower than their (its) lowest effective dose by itself against virus.

17. The composition according to claim 1, wherein the said antiviral agent is an Amantadin type antiviral agent.

18. The composition according to claim 1, wherein the said antiviral agent is a nucleic-acid type antiviral agent.

19. The composition according to claim 1, wherein the said antiviral agent is a peptide type antiviral agent.

20. The composition according to claim 1, wherein the said antiviral agent is a polyanion type antiviral agent.

21. The composition according to claim 1, wherein the said antiviral agent is a phosphoric-acid type antiviral agent.

22. The composition according to claim 1, wherein the said antiviral agent is a endogenous antiviral agent.

23. The composition according to claim 1, wherein the said antiviral agent is a glycoside type antiviral agent.

24. The composition according to claim 1, wherein the said antiviral agent is a lipid type antiviral agent.

25. The composition according to claim 1, wherein the said antiviral agent is a Ansamycin type antiviral agent.

26. The composition according to claim 1, wherein the said antiviral agent is a thiosemicarbazide type antiviral agent.

27. The composition according to claim 18, wherein the said nuclei-acid type antiviral agent is 3'-azido-3'-deoxythymidine (AZT), 2',3'-dideoxythymidine, 2',3'-dideoxyadenosine, 2',3'-dideoxyguanosine, 2',3'-dideoxyinosine, 2',3'-dideoxycytidine, Ara A, Ara C, Ara T, iododeoxyuridine or Acyclovir.

28. The composition according to claim 1, wherein the said compound possessing cell-fusion inhibitor activity and/or virus-adsorption inhibitory activity is a compound having a saccharic carbon atom, said compound being selected from the group consisting of:

a) a natural oligosaccharide,

b) a synthetic oligosaccharide,

c) a natural polysaccharide,

d) a synthetic polysaccharide, and

e) a pharmaceutically acceptable salt of any of the items, a, b, c or d.

and having at least one S-oxoacid group linked to either of its saccharic carbon atoms through a linking group with a low molecular weight.

29. The composition according to claim 28, wherein the said S-oxoacid group is a sulfo group (-$SO_3$ H).

30. The composition according to claim 28, wherein the said linking group is an oxy (-O-) or imino group (-NH).

31. The compound according to claim 28, wherein the said compound is selected from the group consisting of:

a) a sulfated natural polysaccharide,

b) a sulfated synthetic polysaccharide and

c) a pharmaceutically acceptable salt of any of the items, a and b.

32. The composition according to claim 31, wherein the said compound is selected from the group consisting of:

a) a natural polysaccharide having at least one hydrogen sulfate group (-O-$SO_3$ H) which is obtainable from a plant or microoganism,

b) a synthetic polysaccharide having a least one hydrogen sulfate group (-O-$SO_3$ H) produced by esterifying with a sulfating agent a polysaccharide which is obtainable from a plant or microoganism, and

c) a pharmaceutically acceptable salt of any of the items, a and b.

33. The composition according to claim 32, wherein the said compound is a polysaccharide selected from the group consisting of: dextran sulfate, alginic acid sulfate, lentinan sulfate and pullulan sulfate.

34. The composition according to claim 33, wherein the said dextran sulfate has a molecular weight of 500 to 2,000,000.

35. The composition according to claim 34, wherein the said dextran sulfate has a molecular weight of 2,000 to 200,000.

36. The composition according to claim 35, wherein the said dextran sulfate has a molecular weight of 2,000 to 10,000.

37. The composition according to claim 36, wherein the said dextran sulfate has a molecular weight of 3,000 to 8,000.

38. The composition according to claim 33, wherein the said dextran sulfate has a sulfur content of 5 to 23 %.

39. The composition according to claim 32, wherein the said natural polysaccharides include carrageenan or fucoidin.

40. The composition according to claim 31, wherein the said compound is selected from the group consisting of:

a) a natural polysaccharide having at least one sulfo group (-$SO_3$ H) which are obtainable from an animal,

b) a synthetic polysaccharide having at least one sulfo group (-$SO_3$ H) which is produced by esterifying with a sulfating agent a polysaccharide obtained from an animal, and

c) a pharmaceutically acceptable salt of any of the items, a and b.

41. The composition according to claim 40, wherein the said compound is mucopolysaccharides.

42. The composition according to claim 40, wherein the said compound is a sulfated synthetic mucopolysaccharide produced by esterifying a natural mucopolysaccharide with a sulfating agent.

43. The composition according to claim 40, wherein the said compound is a natural polysaccharide selected from the group consisting of heparin, chondroitin sulfate, dermatan sulfate, heparitin sulfate, keratan sulfate, hyaluronic acid, teichuronic acid, chitin, chitosan, mucolipid and mucoprotein or a pharmaceutically acceptable salt thereof.

44. The composition according to claim 40, wherein the said compound is a synthetic polysaccharide selected from the group consisting of chondroitin polysulfate, dermatan polysulfate, heparitin polysulfate, keratan polysulfate, hyaluronic acid sulfate, teichuronic acid sulfate, chitin sulfate and chitosan sulfate or a pharmaceutically acceptable salt thereof.

45. The composition according to claim 1, wherein the said compound possessing cell-fusion inhibitor activity and/or a virus-adsorption inhibitor activity is an antiviral antibody.

46. The composition according to claim 1, wherein the said antiviral agent is 3'-azido-3'-deoxythymidine, and the said compound possessing cell-fusion inhibitory activity and/or virus-adsorption inhibitory activiry is dextran sulfate.

47. The composition according to claim 1, whrein the said antiviral agent is 2',3'-dideoxycytidine, and the said compound possessing cell-fusion inhibitory activity and/or viruc-adsorption inhibitory activiry is dextran sulfate.

48. The composition according to claim 1, wherein the said antiviral agent is 2',3'-dideoxyadenosine, and the said compound possessing cell-fusion inhibitory activity and/or virus-adsorption inhibitory acitiviry is dextran sulfate.

49. The composition according to claim 1, wherein the said disease caused by virus is brought about by retrovirus, herpesvirus or influenza virus.

50. The composition according to claim 49, wherein the said retrovirus is a human or animal retrovirus.

51. The composition according to claim 50, wherein the said human retrovirus is selected from the group consisting of HTLV-I, HTLV-II, HTLV-III, LAV, ARV and the Kawasaki-Disease causative virus.

52. The composition according to claim 50, wherein the said disease is caused by HIV.

53. The composition according to claim 50, wherein the said disease is caused by herpesvirus.

54. The composition according to claim 50, wherein the said disease is caused by influenza virus.

55. The composition according to claim 50, wherein the said animal retrovirus is avian myelobiastosis virus of Friend murine leukemia virus.

56. The composition according to claim 1, wherein the said disease caused by viruses in lymphadenopathy (LAS), persistent generalized lymphadenopathy (PGL), AIDS, AIDS related complex (ARC), adult T-cell leukemia (ATL), kawasaki Disease, herpes or influenza.

57. The use of a compound possessing cell fusion inhibitory activity and/or virus-adsorption inhibitory activity for the manufacture of a medicament for treatment of a disease caused by virus wherein the said medicament is administered with an antiviral agent lacking inhibitory activity against cell-to-cell infection through cell fusion from a virus-infected cell to an uninfected cell and/or inhibitory activity against adsorption of a virus to a target cell.

58. The use according to claim 57, wherein the said antiviral agent is 3'-azido-3'-deoxythymidine, and the said compound possessing cell-fusion inhibitory activity and/or virus-adsorption inhibitory activity is dextran sulfate.

59. The use according to claim 57, wherein the said antiviral agent is 2',3'-dideoxycytidine, and the said compound possessing cell-fusion inhibitory activity and/or virus-adsorption inhibitory activity is dextran sulfate.

60. The use according to claim 57, wherein the said antiviral agent is 2',3'-dideoxyadenosine, and the said compound possessing cell-fusion inhibitory activity and/or virus-adsorption inhibitory activity is dextran sulfate.

61. The use according to claim 57, wherein the said antiviral agent is Ara A, Ara C or Ara T, and the said compound possessing cell-fusion inhibitory activity and/or virus-adsorption inhibitory activity is dextran sulfate.

62. The use according to claim 57, wherein the said antiviral agent is iododeoxyuridine, and the said compound possessing cell-fusion inhibitory activity and/or virus-adsorption inhibitory activity is dextran sulfate.

63. The use according to claim 57, wherein the said antiviral agent is Acyclovir, and the said compound possessing cell-fusion inhibitory activity and/or virus-adsorption inhibitory activity is dextran sulfate.

64. The composition according to claim 1, wherein said compound possessing cell fusion inhibitory activity and/or virus-adsorption inhibitory activiry is a combination of 2 or more compounds.

65. The use according to claim 57, wherein the antiviral agent and the compound possessing cell fusion inhibitory activity and/or virus-adsorption inhibitory activiry are administered simultaneously and/or sequentially.

Fig. 1

0 270 317

Fig. 2

0 270 317

# Fig. 3

0 270 317

# Fig. 4

0 270 317

Fig. 5

Fig. 6

Fig. 7

Fig. 8

0 270 317

Fig. 9

## Fig. 10

0 270 317

Fig. II

0 270 317

Fig. 12

Fig. 13

Fig. 14